# EUROPÄISCHE PATENTSCHRIFT

(11) **EP 1 248 554 B1**
(45) Veröffentlichungstag und Bekanntmachung des Hinweises auf die Patenterteilung: **13.04.2005**
(21) Anmeldenummer: 00989938.6
(22) Anmeldetag: 01.12.2000
(51) Int. Cl.: A61B 3/10, A61B 3/02

(54) **VORRICHTUNG UND VERFAHREN ZUR BESTIMMUNG VON KORREKTURDATEN FÜR DIE KORREKTUR DER ABERRATIONEN EINES AUGES**
DEVICE AND METHOD FOR DETERMINING THE CORRECTION DATA FOR CORRECTING ABERRATIONS OF THE EYE
DISPOSITIF ET PROCEDE DE DETERMINATION DE DONNEES DE CORRECTION PERMETTANT LA CORRECTION DES ABERRATIONS D'UN OEIL

(30) Priorität: 03.12.1999 DE 19958436
(43) Veröffentlichungstag der Anmeldung: 16.10.2002
(73) Patentinhaber: Asclepion-Meditec AG, 07745 Jena (DE)
(72) Erfinder: DICK, Manfred, 07926 Gefell (DE); MÄUSEZAHL, Holger, 07745 Jena (DE); SCHRÖDER, Eckhard, 90542 Eckental (DE)
(74) Vertreter: Schnekenbühl, Robert Matthias L.
(86) Internationale Anmeldenummer: PCT/EP2000/012116
(87) Internationale Veröffentlichungsnummer: WO 2001/039660

(56) Entgegenhaltungen:
- WO-A-93/22711
- US-A- 4 579 430
- US-A- 5 777 719
- LIANG ET AL: 'Supernormal Vision and High Resolution Retinal Imaging through Adaptive Optics' J. OPT. SOC. AM. Bd. 14, Nr. 11, November 1997, Seiten 2884 - 2892

## Beschreibung

Die Erfindung betrifft eine Vorrichtung und ein Verfahren zur Bestimmung von Korrekturdaten für die Aberrationen eines Auges. Insbesondere betrifft die Erfindung ein Meßsystem zur aktiven, physiologisch bewerteten Ermittlung der gesamten Aberrationen des menschlichen Auges, dessen Meßdaten zur optimalen, vorzugsweisen Laser-Korrektur der Aberrationen für die Realisierung eines bestmöglichsten Sehvermögens des menschlichen Auges dienen.

Für die Korrektur von Sehfehlern ist es eine ursprüngliche Methode, daß der Arzt zur Korrektur von Myopie, Hyperopie und Astigmatismus den Patienten entsprechende Linsen in einem Brillengestell zum Sehtest anbietet, und der Patient mit Hilfe einer Sehtafel sein im Rahmen der Abstufungen der Testlinsen optimales Sehvermögen feststellen kann. Dementsprechend kann der Patient eine optimale Brille zur Korrektur grober Sehfehler erhalten.

Dieses klassische Herangehen wurde mittlerweile durch objektivierte Meßmethoden ohne aktive Mitwirkung des Patienten abgelöst. Beispiele hierfür sind automatische Refraktometer.

Neben der objektiven Ermittlung der groben Sehfehler setzt man gegenwärtig verschiedene Topografiegeräte und Aberroskope ein, um patientenspezifische Hornhauttopografien und auch die gesamten Wellenfrontaberrationen zu ermitteln. Auf Basis dieser Meßdaten werden den Patienten zum Beispiel mit Hilfe eines Excimerlasersystems spezifische Hornhauttopografien appliziert, welche ein optimales Sehvermögen garantieren sollen (vgl. P. Mierdel, H.-E. Krinke, W. Wiegand, M. Kaemmerer, T. Seiler "Meßplatz zur Bestimmung der monochromatischen Aberration des menschlichen Auges" Ophthalmologe 1997, 94; 441-445, Springer Verlag 1997).

Bei diesen Messungen muß aus technischen Gründen ein doppelter Durchgang durch das optische System des Auges realisiert werden. Dabei ist das Hauptproblem, daß die ungeraden Aberrationen verfälscht bestimmt werden. Dieses Problem kann beim doppelten Durchgang zum Beispiel dadurch reduziert werden, daß man unterschiedliche numerische Aperturen für das ein- und austretende Licht realisiert. Ein anderer Ansatz ist die Nutzung einer auf der Netzhaut induzierten Fluoreszenz um diese Meßfehler auszuschließen (vgl. Laser Focus World, April 1999, 35-36).

Bei den Methoden des Standes der Technik wird lediglich ein an sich dynamischer optischer Apparat in einer Momentaufnahme genau vermessen und korrigiert. Dies impliziert insbesondere bei der gewünschten Korrektur von Aberrationen höherer Ordnung Fehler, die das optimale Sehvermögen nicht erreichen lassen. Dies wird dadurch belegt, daß die Messung der Aberrationen auch bei Augen mit bestem Sehvermögen manchmal erstaunlich hohe Aberrationen zeigen und niemand heute sicher weiß, ob eine physikalische Korrektur des Auges das Sehvermögen steigert oder gar verschlechtert.

Bekannt ist weiterhin, daß man mit Hilfe einer adaptiven Optik die Aberrationen eines menschlichen Auges kompensieren kann, um hochaufgelöste Aufnahmen der Retina für medizinische Untersuchungen zu realisieren (vgl. Laser Focus World, August 1998, 18-22).

In der DE 19733193 A1 wird ein Mikroskop mit adaptiver Optik beschrieben. Hierbei werden verschiedene Wellenmodulatoren erwähnt.

In dem Aufsatz "Supernormal vision and high-resolution retinal imaging through adaptive optics" von Liang et al., J. Opt. Soc. Am. A, Vol 14 (1997). S. 2884-2892 wird eine Vorrichtung und ein Verfahren beschrieben, bei dem mittels einer Wellenfrontmessung unter Zuhilfenahme eines deformierbaren Spiegels eine adaptive Kompensation der Aberration eines Auges sowie photographische Aufnahmen der Retina möglich sind.

Die US 5,777,719 beschreibt ebenfalls ein Verfahren und eine Vorrichtung zur verbesserten Aufnahme von Photographien der Retina, wobei die Vorrichtung unter Nutzung eines deformierbaren Spiegels korrigierte Bilder der Retina mittels einer CCD-Kamera aufnehmen kann.

Aufgabe der vorliegenden Erfindung ist es daher, ein Verfahren und eine Vorrichtung bereitzustellen, mit denen Korrekturdaten für die Korrektur der Aberrationen eines Auges bestimmt werden können, deren Umsetzung zu verbesserten Sehbedingungen beim Patienten führen.

Diese Aufgabe wird durch die Vorrichtung und das Verfahren nach den unabhängigen Ansprüchen gelöst. Vorteilhafte Ausgestaltungen der Erfindung sind in den Unteransprüchen angegeben.

Insbesondere wird die Aufgabe durch eine Vorrichtung zur Bestimmung von Korrekturdaten für die Korrektur der Aberrationen eines Auges eines Patienten gelöst, das ein optisches System umfaßt, wobei die Vorrichtung weiterhin eine adaptive Optik bzw. einen Wellenfrontmodulator, eine Einrichtung zur Darstellung von Testbildern mittels der adaptiven Optik und eine Steuereinrichtung umfaßt. Durch diese Vorrichtung ist es möglich, den Patienten Testbilder mittels der adaptiven Optik einzuspielen, die dieser subjektiv bewerten kann. Durch die subjektive Bewertung des Testbildes auf der adaptiven Optik können kleinste Aberrationen des Auges erkannt werden und die subjektiv vom Patienten als beste Kompensation empfundene Korrektur ermittelt werden. Die Einrichtung der adaptiven Optik in dem Augenblick, in dem die Korrektur vom Patienten als subjektiv beste empfunden wird, korrespondiert dann zu den Korrekturdaten für die Korrektur der Aberration des Auges.

Das optische System dient dazu, um den Strahlengang vom Auge zur adaptiven Optik auszurichten bzw. zu focusieren. Ein solches optisches System kann beispielsweise eine Linsenanordnung sein.

Die adaptive Optik ist bevorzugt ein Modulator, insbesondere ein Wellenfrontmodulator. Diese sind in verschiedenen Ausführungen denkbar. So sind etwa transmittierende Modulatoren auf LCD-Basis erhältlich oder reflektierende Modulatoren mit beweglichen Membranen. Diese wiederum können nach der Art ihrer Stellelemente unterschieden werden: piezo-gesteuert, elektrostatische und bimorphe Membranen. Besonders bevorzugt werden als adaptive Optik elektrostatische Membranspiegel oder hierzu elektrisch gesteuerte Mikrospiegelfelder eingesetzt. Als adaptive Optik können sowohl reflektierende als auch transmittierende Medien benutzt werden.

Die Einrichtung zur Darstellung von Testbildern mittels der adaptiven Optik kann bei reflektierenden Elementen der adaptiven Optik ein Projektor sein, der diese Testbilder auf die reflektierenden Elemente der adaptiven Optik projiziert, die dann bevorzugt über das optische System an das Auge gelangen. Bei in Transmission arbeitender adaptiver Optik wird ein Projektor vorgesehen, der durch die adaptive Optik hindurch das Bild über das optische System auf das Auge appliziert. Es ist auch denkbar bei adaptiver Optik mit LED-Anzeige als Einrichtung zur Darstellung von Testbildern elektrische Signale zu verwenden, die auf den einzelnen LED-Flächen entsprechende Testbilder bzw. Teile dieser Testbilder darstellen.

Die Testbilder können bevorzugt so ausgebildet sein, daß sie bezüglich der zu untersuchenden Abberationen besonders deutlich auf die modulierte Wellenfront durch die adaptive Optik reagieren. So kann bei dem Testbild ein Bildfehleranteils scannend durchfahren werden und die Wellenfront so deformiert werden, daß nur ein einziger Bildfehler verändert wird. Durch das vom Patienten iterativ bestimmte subjektiv verzeichnungsfreie Testbild wird in Überlagerung zur Wellenfrontaberration des Auges ein subjektiv aberrationsfreies Bild auf dem Auge erzeugt.

Die Steuereinrichtung besteht bevorzugt aus einem Computer. Mittels der Steuereinrichtung kann bevorzugt die adaptive Optik gesteuert werden sowie die Korrekturdaten für die Korrektur der Aberrationen eines Auges bestimmt werden. Besonders bevorzugt handelt es sich bei der Steuereinrichtung um einen Rechner, der n x m mikrooptische Elemente ansteuert, wobei jedes Element einzeln ansteuerbar bezüglich Kippwinkel und Höhenverstellung ist. Damit kann die Wellenfront lokal definiert geändert werden.

Die Korrekturdaten werden bevorzugt verwendet, um ein optimales, patientenspezifisches refraktives Element zu ermitteln. Als refraktive Elemente kommen insbesondere bevorzugt IOL's, ICL's, Kontaktlinsen oder Brillengläser in Betracht. Besonders bevorzugt werden die Korrekturdaten zur Ermittlung der Schußpositionen einer geplanten Laserbehandlung des Auges, insbesondere bevorzugt der Cornea, verwendet.

Bevorzugt besteht die adaptive Optik aus einer Vielzahl von einzelnen positionierbaren Spiegeln. Diese einzelnen Spiegel können nach dem subjektiven Empfinden des Patienten so eingestellt werden, daß das dargestellte Testbild vom Patienten subjektiv optimal wahrgenommen wird. So können mit Hilfe der adaptiven Optik, der ausgewählten Testbilder und entsprechenden Bewertungsalgorithmen die aktiv physiologisch bewerteten Spiegelpositionen in Korrekturdaten für eine Korrektur der Aberrationen eines Auges zur Herstellung eines optimalen Sehvermögens eines Patienten überführt werden. Die Gesamtheit der Spiegelstellungen definiert dabei ein Datenset, das die subjektiv als optimal empfundene Korrektur aufgrund der adaptiven Optik beschreibt: die Adaptivdaten.

Besonders bevorzugt ist die adaptive Optik als Kugelsegment ausgebildet. Dadurch ist es möglich, die Korrekturdaten aus den Normalen der positionierten Spiegel zu bestimmen, da die adaptive Optik als Kugelsegment direkt zur Außenform des Auges korrespondiert. Auf diese Weise werden ansonsten erforderliche Umrechnungen der gewonnenen Parameter zu den Korrekturdaten eingespart.

In ein besonders bevorzugten Ausführungsbeispiel der vorliegenden Erfindung ist eine Steuereinrichtung vorgesehen, durch die die adaptive Optik einrichtbar ist. Auf diese Weise kann durch Veränderung der einzelnen Spiegel jegliches Aberrationsverhalten eines Augen kompensiert werden und die so dargestellten Testbilder für speziell vorwählbare Aberrationen im menschlichen Auge aufbereitet werden. Durch diese Steuereinrichtung können auch vorbestimmte Sets von Aberrationen höherer Ordnung dargestellt und im Dialog mit dem Patienten ausgewertet werden. Insbesondere bevorzugt kann mittels der Steuereinrichtung die adaptive Optik im Scan-Betrieb eine Serie von Aberrationen des Auges durchtesten und so die optimale Einstellung der adaptiven Optik für ein optimales Sehvermögen des Patienten ermitteln.

Bevorzugt umfaßt ein Ausführungsbeispiel der vorliegenden Erfindung weiterhin eine Aberrationsmeßeinrichtung, insbesondere ein Aberrationsmeßgerät, zur Bestimmung von Aberrationsdaten, die zur objektiven Aberration des Auges korrespondieren. Solche Aberrationsmeßeinrichtungen, beispielsweise Refraktometer oder Aberroskope bzw. Wellenfrontanalysevorrichtungen, können die objektiven Aberrationen des Auges bestimmen und zur Überprüfung der berechneten Korrekturwerte des Patienten durch denselben über eine adaptive Optik bestätigt oder korrigiert werden. Hierdurch ist eine Kombination der subjektiv gewonnenen Korrekturwerte mit Hilfe einer adaptiven Optik mit objektiv ermittelten Werten eines Aberroskopes vorteilhaft vereint.

Besonders bevorzugt werden die Korrekturdaten für die Korrektur der Aberration des Auges durch eine zweite Steuereinrichtung bestimmt. Diese zweite Steuereinrichtung ist bevorzugt ein Computer. Mittels entsprechender Software kann auf der Grundlage der Adaptivdaten beziehungsweise Aberrationsdaten der Satz an Korrekturdaten ermittelt werden. Bevorzugt werden die objektiven Aberrationsdaten als grobe Werte ermittelt, um auf dieser Grundlage durch eine subjektive Bewertung der Testbilder mittels der adaptiven Optik die Adaptivdaten zu bestimmen und diese dann als Basis für die Korrekturdaten zu verwenden. Es ist bevorzugt auch denkbar, den Mittelwert der Adaptivdaten und der Aberrationsdaten als Grundlage zur Bestimmung der Korrekturdaten zu verwenden.

Die Aufgabe der vorliegenden Erfindung wird insbesondere auch durch ein Verfahren zur Bestimmung von Korrekturdaten für die Korrektur der Aberration eines Auges gelöst, bei dem in einem ersten Schritt mittels einer adaptiven Optik Adaptivdaten über eine subjektive optimale Korrektureinstellung des Auges ermittelt werden und bei dem in einem weiteren Schritt die Korrekturdaten für die Korrektur des Auges auf der Basis der Adaptivdaten ermittelt werden. Vorteilhaft an diesem Verfahren ist, daß die Korrekturdaten für die das Sehvermögen negativ beeinflussenden Abbildungsfehler des Auges nunmehr in einem vorab aktiv physiologisch bewerteten Meßverfahren gewonnen werden. Damit ist man nicht mehr auf eine mit Meßfehlern behaftete Ermittlung von objektiven Abbildungsfehlern in einem dynamisch-organischen Abbildungsapparat angewiesen, bei der keine Eigenschaften der Signalverarbeitung durch das menschliche Nervensystem, beziehungsweise Gehirn, einbezogen werden. Im Dialog mit dem Patienten werden hierbei mit Hilfe der adaptiven Optik die Werte ermittelt, die den subjektiv optimalen Korrekturzustand darstellen - dieser kann von dem objektiv als optimal ermittelten Zustand abweichen.

In einem bevorzugten Verfahren der vorliegenden Erfindung werden zusätzlich Aberrationsdaten ermittelt, die zu den objektiven Aberrationen des Auges korrespondieren und die Korrekturdaten werden auf der Basis der Adaptivdaten und der Aberrationsdaten ermittelt. Durch einen Vergleich der subjektiv bewerteten Daten sowie der objektiv ermittelten Daten ist es möglich, die physiologischen Einflüsse der Bildverarbeitung deutlich zu machen.

Besonders bevorzugt werden mit Hilfe objektiver Meßmethoden durch die Aberrationsmeßeinrichtungen bzw. durch die genannten Meßgeräte die groben Korrekturwerte bestimmt und als Ausgangswerte für die weitere Bestimmung der endgültigen Korrekturwerte durch das subjektive, d.h. aktive, physiologisch bewertete Verfahren bestimmt.

Bei einem bevorzugten Verfahren der vorliegenden Erfindung werden die Adaptivdaten für eine subjektiv optimale Korrektureinstellung des Auges durch eine subjektive Beurteilung von durch Veränderung der Spiegelstellung der Spiegel der adaptiven Optik modifizierten Testbildern durch den Patienten gewonnen. Durch die Veränderung der Spiegelstellung der einzelnen kleinen Spiegel ist es möglich, jegliches Aberrationsverhalten des Auges zu kompensieren. Die Stellung der einzelnen kleinen Spiegelchen wird daraufhin in der Stellung erfaßt, die für den Patienten das Optimum der Kompensation der Aberration darstellen. Die Positionsparameter der einzelnen kleinen Spiegel entsprechen damit der subjektiv als optimal empfundenen Korrektur der Aberration des Auges. Diese Positionsparameter der Spiegel sind einfach zu erfassen und bilden eine gut verwertbare Grundlage zur Ermittlung der Adaptivdaten bzw. der Korrekturdaten.

Ausführungsbeispiele der Erfindung und vorteilhafte Ausgestaltungen sollen im Folgenden anhand von Zeichnungen näher erläutert werden. Dabei zeigen:
Fig. 1 ein Blockschaltbild eines Ausführungsbeispieles der erfindungsgemäßen Vorrichtung;
Fig. 2a ein Spiegel einer adaptiven Optik;
Fig. 2b einen Auschnitt aus einem Feld von Spiegeln gemäß Figur 2a, die eine adaptive Optik bilden;
Fig. 3 ein Flußdiagramm zur Darstellung eines Ausführungsbeispiels des erfindungsgemäßen Verfahrens;
Fig. 4a eine Darstellung von einem ersten Testbild mit korrespondierender adaptiver Fläche und Wellenfront in drei Zuständen;
Fig. 4b eine Darstellung von einem zweiten Testbild mit korrespondierender adaptiver Fläche und Wellenfront in drei Zuständen; und
Fig. 4c eine Darstellung von einem dritten Testbild mit korrespondierender adaptiver Fläche und Wellenfront in drei Zuständen;

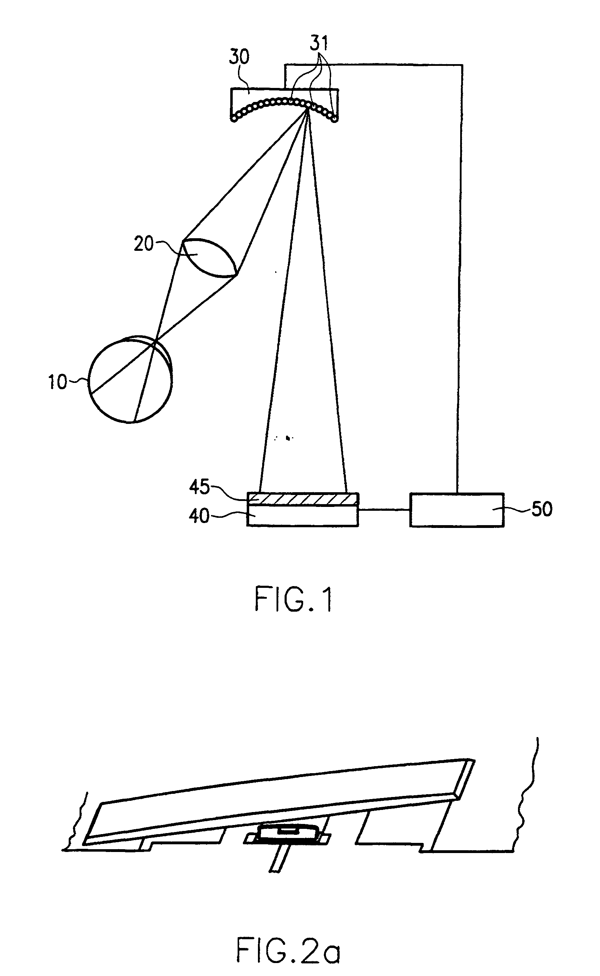

**Figur 1** zeigt ein Blockschaltbild eines Ausführungsbeispieles der erfindungsgemäßen Vorrichtung. Das Auge 10 betrachtet über eine Linse 20 und ein Spiegelfeld einer adaptiven Optik 30, die aus einzelnen Spiegeln 31 besteht. Eine Einrichtung zur Darstellung von Testbildern 40 erzeugt wahlweise Testbilder 45. Eine Steuereinrichtung 50 ist sowohl mit der Einrichtung zur Darstellung von Testbildern 40 als auch mit der adaptiven Optik 30 verbunden.

Das Testbild 45 wird mittels der Einrichtung zur Darstellung von Testbildern 40 erzeugt und auf die adaptive Optik 30 projiziert. Die einzelnen Spiegel 31 projizieren das Testbild über die Linse 20 auf die Netzhaut des Auges 10. Die Steuereinrichtung 50 erfaßt dabei den Zustand der einzelnen Spiegel 31 und die Auswahl des Testbildes 45.

Der Patient beurteilt nun die subjektive Qualität des Abbildes des auf der Netzhaut des Auges 10 erfaßten Testbildes 45, bevorzugt indem er das Testbild als gut bestätigt oder als schlecht verwirft. Vermutet er Korrekturbedarf, dann wird über die Steuereinheit 50 die Stellung der einzelne Spiegel 31 so lange modifiziert, bis das Abbild des Testbildes auf der Netzhaut des Auges 10 optimal erscheint. Die Stellung der einzelnen Spiegel 31 wird dann als Wertetupel für die Adaptivdaten für dieses Testbild 45 übernommen und korrespondiert zur vom Patienten erwünschten, subjektiv als optimal empfundenen Korrektur der Aberrationen für das betrachtete Testbild 45. Besonders bevorzugt kann dieses Nachführen der einzelnen Spiegel 31 bis zur subjektiv optimalen Position im Scanbetrieb erfolgen. Dabei werden dem Patienten mit Hilfe von nach einzelnen Bildfehlern separierten Bewertungsalgorithmen Toleranzfelder dieser Bildfehler im Scanbetrieb vorgeführt und die optimale Einstellung der adaptiven Opik ermittelt.

**Figur 2a** zeigt einen einzelnen Spiegel 31 einer adaptiven Optik 30. Dieser Spiegel weist eine etwa quadratische Grundfläche auf. Die Kantenlänge des Spiegls 31 mißt ca. 100 µm. Dieser Spiegel 31 ist bevorzugt monolithisch über einem Schaltkreis aus herkömmlichen CMOS SRAM Zellen hergestellt. Der Spiegel 31 wird durch elektrostatische Kräfte betätigt, die dadurch erzeugt werden, daß zwischen der Spiegelplatte und der Elektrode eine Spannungsdifferenz angelegt wird. Bevorzugt werden auch piezoelektrische Effekte genutzt, um die Position des einzelnen Spiegels festzulegen. Diese Spiegel 31 in der adaptiven Optik 30 sind geeignet, um hohe Kippfehler auszugleichen.

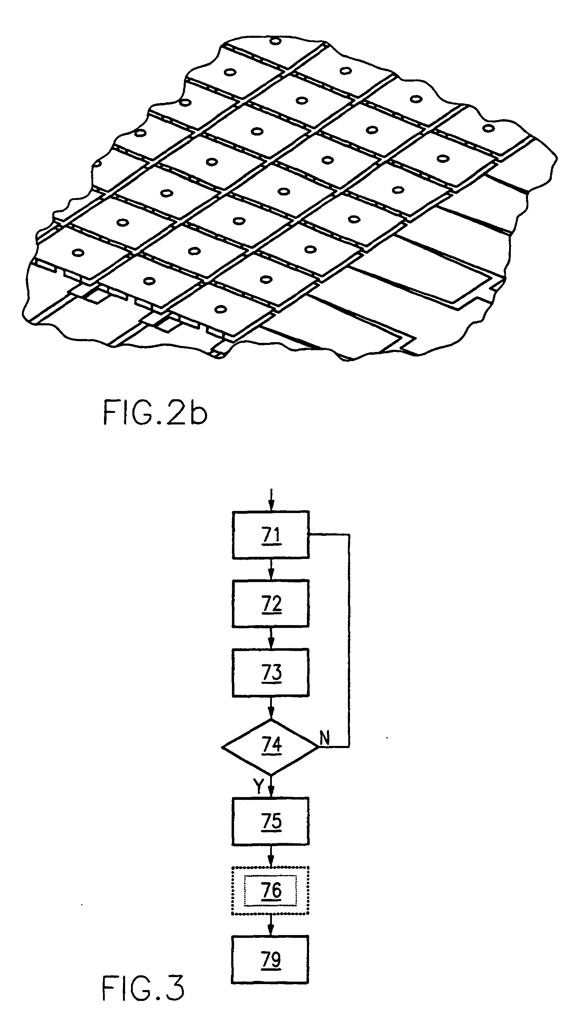

**Figur 2b** zeigt einen Auschnitt aus einem Feld von Spiegeln 31 gemäß Figur 2a, die eine adaptive Optik 30 bilden. Spiegel 31 sind Kante an Kante in einem Spiegelfeld ausgerichtet und bilden so eine zusammenhängende Spiegelfläche, bei der jeder einzelne kleine Spiegel 31 individuell ausrichtbar ist. Damit können auch Aberrationen des Auges 10 höherer Ordnung ausgeglichen werden. Die Positionen der einzelnen Spiegel 31 im als vom betrachtenden Patienten als optimal empfundenen Zustand als Wertetupel entsprechen dann den Adaptivdaten. Diese Konstellation der adaptiven Optik, d.h. die Stellung der Gesamtheit aller einzelnen Spiegel 31, wird an die Steuereinrichtung 50 übergeben und in die Korrekturdaten umgesetzt. Besonders bevorzugt weist die adaptive Optik, d.h. das Spiegelfeld, eine Kontur auf, die der Hornhautoberfläche des Auges 10 entspricht. Dies ist beispielsweise ein Kugelsegment. Dadurch können die Normalen der einzelnen Spiegel 31 als Adaptivdaten übernommen werden, so daß eine Umrechnung der einzelnen Daten der Spiegelstellungen vereinfacht werden kann.

**Figur 3** zeigt ein Flußdiagramm zur Darstellung eines Ausführungsbeispiels des erfindungsgemäßen Verfahrens. Mit diesem bevorzugten Verfahren 70 wird in Schritt 71 ein Testbild geladen, das über die Einrichtung zur Darstellung von Testbildern 40 über die adaptive Optik 30 auf das Auge 10 abgebildet wird. Nun wird in einem Schritt 72 die adaptive Optik 30 für dieses Testbild 45 so lange geändert, bis die subjektiv optimale Einstellung aufgefunden wurde. Diese subjektiv als optimal empfundene Einstellung wird dann in Schritt 73 ermittelt und gespeichert. In Schritt 74 wird abgefragt, ob die optimale Wellenfrontkorrektur bereits erreicht wurde, oder ob noch weitere Aberrationen zu korrigieren sind. Dies kann aufgrund einer Einschätzung des Patienten erfolgen oder aufgrund einer abzuarbeitenden Serie von vorbestimmten Testbildern entschieden werden, die geeignet sind, um spezielle Abbildungsfehler aufzuspüren. Sobald keine Testbilder mehr abzuarbeiten sind, werden in Schritt 75 die Adaptivdaten aus den verschiedenen gewonnenen Spiegelstellungen der adaptiven Optik 30 berechnet. Bevorzugt geschieht dies durch ein Verfahren nach dem Zernike Polynom oder dem Taylor Polynom.

Bevorzugt werden aus den Adaptivdaten 35 dann in Schritt 79 die Korrekturdaten 55 bestimmt. Diese werden besonders bevorzugt zur Ermittlung der Schußkoordinaten für ein Lasersystem 60 zur Korrektur des Auges 10 verwendet

In einem weiteren bevorzugten Ausführungsbeispiel des erfindungsgemäßen Verfahrens wird in einem Schritt 76 über eine objektive Meßmethode auch über eine Aberrationsmeßeinrichtung bzw. ein Aberroskop 80 Aberrationsdaten 85 ermittelt und auch diese bei der Bestimmung der Korrekturdaten 55 in Schritt 79 berücksichtigt.

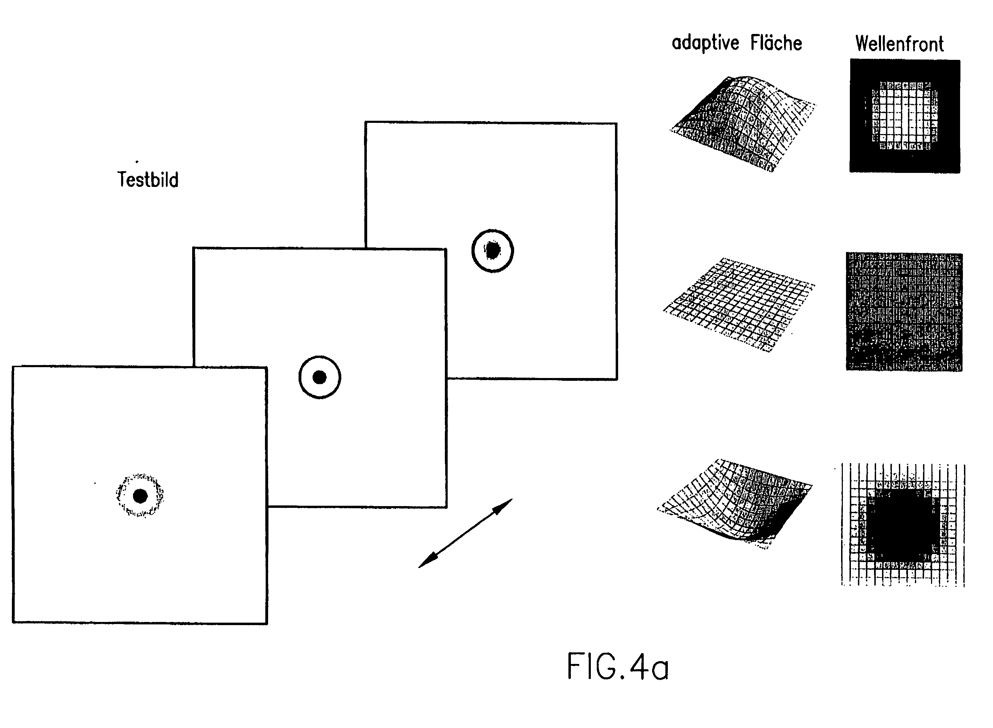

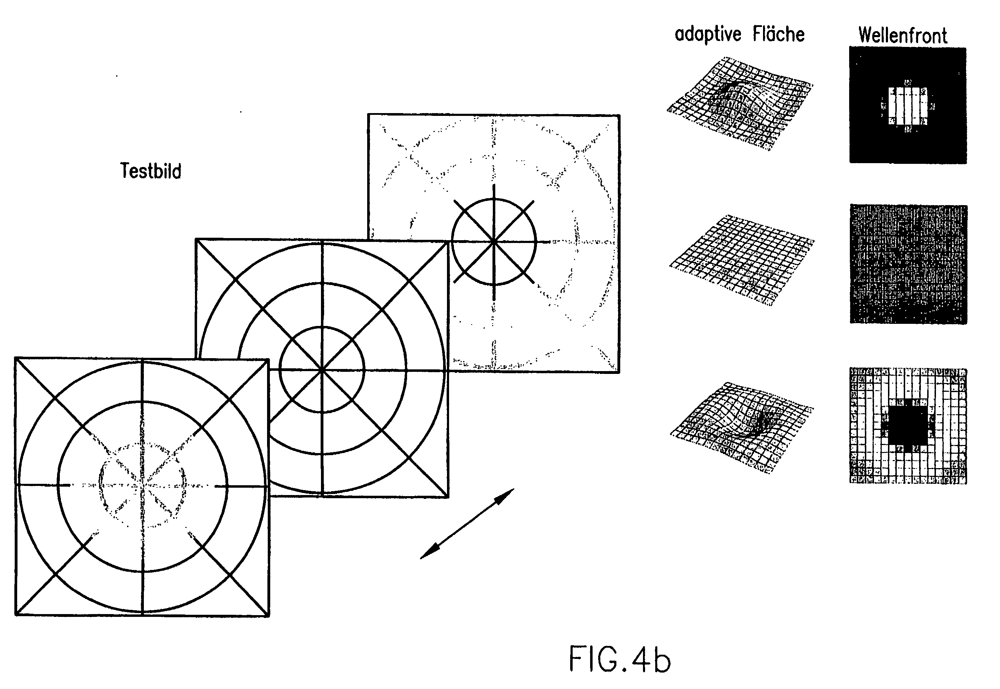

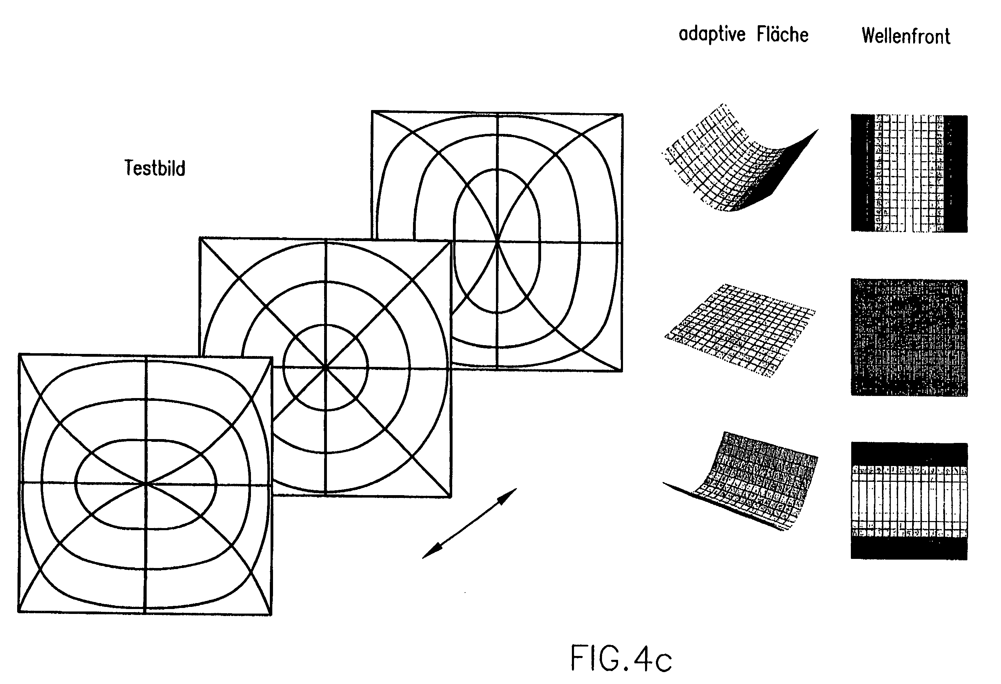

In den **Figuren 4 a, b und c** sind Beispiele von drei verschiedenen Testbildern mit korrespondierender adaptiver Fläche und Wellenfront in drei Zuständen dargestellt. In den drei Zeilen der einzelnen Figuren sind Testbilder in verschiedenen deformierten Zuständen dargestellt. In der linken Spalte ist das Testbild dargestellt, wie es mit einem aberrationsfreien Auge wahrgenommen würde. In der mittleren Spalte ist die Geometrie der adaptiven Fläche dargestellt, die eine solche Verzeichnung des Testbildes für das aberrationsfreie Auge hervorrufen würde und in der rechten Spalte ist mit abgestuften Grauwerten in einer Matrix die korrespondierende Wellenfront graphisch dargestellt.

Bei dem Testbild in **Figur 4 a** ist ein zentraler Punkt und ein darum zentrierter Ring dargestellt. In der Abbildung in der mittleren Zeile ist die adaptive Optik planar ausgerichtet, wie dies in der mittleren Spalte der mittleren Zeile zu erkennen ist, und moduliert die Wellenfront nicht - dies kann in der Darstellung der Wellenfront in der rechten Spalte gut erkannt werden. Das Testbild in der linken Spalte erscheint daher unverzeichnet und klar. In der oberen Zeile weist die adaptive Fläche ein lokales Maximum im Zentrum auf. Daher ist die Wellenfront in der Mitte verschoben und das Testbild wird im Zentrum verzeichnet wahrgenommen. Bei einem Betrachter, dessen Auge eine entsprechend anders orientierte Aberration aufweist, wird dieses Bild dann als verzeichnungsfrei erkennen, so daß seine Aberration hierdurch erkannt wird. In der untersten Zeile weist die adaptive Fläche ein lokales Minimum im Zentrum auf, so daß eine entsprechende andere Aberration ausgeglichen wird.

Durch die Ansteuerung der adaptiven Optik derart, daß ein Bildfehleranteil scannend duchfahren wird - also beispielsweise von der adaptiven Fläche in der ersten Zeile über die zweite zur dritten Zeile und zurück - wird die Wellenfront so deformiert, daß nur ein Bildfehler verändert wird. Der Patient sucht nun iterativ das subjektiv verzeichnungsfreie Testbild. In Überlagerung zur Wellenfrontaberration des Auges entsteht so ein subjektiv aberrationsfreies Bild. Auf diese Weise kann aus der Form der adaptiven Fläche bzw. der hierzu korrespondierenden Wellenfront auf die Aberration des Auges des Patienten geschlossen werden.

In den **Figuren 4 b und 4 c** sind weitere Testbilder für andere Bildfehler dargestellt. Hierfür sind dann entsprechende modifizierte adaptive Flächen und korrespondierende Wellenfronten angegeben. Es gilt hierfür entsprechend das Gleiche, wie für das Testbild der Figur 4 a.

Mit der vorliegenden Erfindung ist daher eine Vorrichtung und ein Verfahren zur Bestimmung von Korrekturdaten für die Korrektur der Aberrationen eines Auges bereitgestellt worden, welche nicht nur die Aberrationen des optischen Abbilungsapparates berücksichtigt, sondern auch die Empfängereigenschaften und Signalverarbeitung im menschlichen Gehirn. Ein besonderer Vorteil liegt darin, daß die Korrekturdaten für die das Sehvermögen neagtiv beeinflussenden Abbildungsfehler des menschlichen Auges nunmehr in einem vorab aktiv physiologisch bewerteten Meßverfahren gewonnen werden.

### Bezugszeichenliste

### Verfahren und Vorrichtung zur ...

- 10.: Auge
- 20.: optisches System
- 30.: adaptive Optik
- 31.: Spiegel
- 35.: Adaptivdaten
- 40.: Einrichtung zur Darstellung von Testbildern
- 45.: Testbild
- 50.: Steuereinrichtung
- 51.: erste Steuereinrichtung (adaptive Optik)
- 52.: zweite Steuerreinrichtung (Berechnung Korrekturdaten)
- 55.: Korrekturdaten
- 60.: Lasersystem
- 80.: Aberrationsmeßeinrichtung bzw. Aberroskop
- 85.: Aberrationsdaten

## Patentansprüche

1. Vorrichtung (1) zur Bestimmung von Korrekturdaten (55) für die Korrektur der Aberrationen eines Auges (10) eines Patienten umfassend ein optisches System (20);
eine adaptive Optik (30);
eine Einrichtung zur Darstellung von Testbildern (40), die mittels der adaptiven Optik (30) und das optischen Systems (20) in das Auge projiziert werden, und
eine Steuereinrichtung (50) für die adaptive Optik, womit der Projektion der Testbilder auf der Basis subjektives Angaben des Patienten gesteuerte Aberrationen aufgeprägt werden können, die die Aberrationen des Auges kompensieren.

2. Vorrichtung (1) nach Anspruch 1
**dadurch gekennzeichnet,**
**daß** die adaptive Optik (30) aus einer Vielzahl von einzelnen positionierbaren Spiegeln (31) besteht.

3. Vorrichtung (1) nach einem der vorhergehenden Ansprüche
**dadurch gekennzeichnet,**
**daß** die adaptive Optik (30) als Kugelsegment ausgebildet ist.

4. Vorrichtung (1) nach einem der vorhergehenden Ansprüche
**dadurch gekennzeichnet,**
**daß** die adaptive Optik (30) durch eine erste Steuereinrichtung (51) einrichtbar ist.

5. Vorrichtung (1) nach einem der vorhergehenden Ansprüche
**dadurch gekennzeichnet,**
**daß** die Vorrichtung (1) weiter umfaßt:
eine Aberrationsmeßeinrichtung (80) zur Bestimmung von Aberrationsdaten (85), die zur objektiven Aberration des Auges (10) korrespondieren.

6. Vorrichtung (1) nach einem der vorhergehenden Ansprüche
**dadurch gekennzeichnet,**
**daß** die Korrekturdaten (55) für die Korrektur der Aberrationen des Auges (10) durch eine zweite Steuereinrichtung (52) auf der Grundlage von Adaptivdaten (35), die zur adaptiven Optik korrespondieren oder
der Adaptivdaten (35) und der Aberrationsdaten (85) bestimmbar sind.

7. Verfahren zur Bestimmung von Korrekturdaten (55) für die Korrektur der Aberrationen eines Auges (10)
**dadurch gekennzeichnet,**
**daß** in einem ersten Schritt (73) mittels einer Vorrichtung nach Anspruch 1 Adaptivdaten (35) für eine subjektiv optimale Korrektureinstellung des Auges (10) ermittelt werden und in einem weiteren Schritt (79) die Korrekturdaten (55) für die Korrektur des Auges (10) auf der Basis der Adaptivdaten (35) ermittelt werden.

8. Verfahren nach dem vorhergehenden Anspruch
**dadurch gekennzeichnet,**
**daß** zusätzlich Aberrationsdaten (85) ermittelt werden, die zur objektiven Aberration des Auges (10) korrespondieren und die Korrekturdaten (55) auf der Basis der Adaptivdaten (35) und der Aberrationsdaten (85) ermittelt werden.

9. Verfahren nach einem der vorhergehenden Verfahrensansprüchen,
**dadurch gekennzeichnet,**
**daß** die Adaptivdaten (35) für eine subjektiv optimale Korrektureinstellung des Auges (10) durch eine subjektive Beurteilung von durch Veränderung der Spiegelstellung der Spiegel (31) der adaptiven Optik (30) modifizierten Testbildern (45) durch den Patienten gewonnen werden.

## Claims

1. Device (1) for determining correction data (55) for the correction of the aberrations of an eye (10) of a patient, comprising an optical system (20); an adaptive lens system (30);
an apparatus for displaying test patterns (40) which are projected into the eye by means of the adaptive lens system (30) and the optical system (20), and a control apparatus (50) for the adaptive lens system with which controlled aberrations which compensate for the aberrations of the eye can be impressed on the projection of the test patterns on the basis of subjective data provided by the patient.

2. Device (1) according to claim 1
**characterized in that**,
the adaptive lens system (30) is composed of a large number of individual positionable mirrors (31).

3. Device (1) according to one of the previous claims,
**characterized in that**
the adaptive lens system (30) is developed as a sphere element.

4. Device (1) according to one of the previous claims,
**characterized in that**
the adaptive lens system (30) can be adjusted by a first control apparatus (51).

5. Device (1) according to one of the previous claims,
**characterized in that**
the device (1) also comprises:
an aberration-measuring apparatus (80) for determining aberration data (85) which correspond to the objective aberration of the eye (10).

6. Device (1) according to one of the previous claims,
**characterized in that**
the correction data (55) for the correction of the aberrations of the eye (10) can be determined by a second control apparatus (52) on the basis of adaptive data (35) which correspond to the adaptive lens system or on the basis of the adaptive data (35) and the aberration data (85).

7. Method for determining correction data (55) for the correction of aberrations of an eye (10),
**characterized in that**
in a first step (73), by means of a device according to claim 1, adaptive data (35) are established for a subjectively optimum correction adjustment of the eye (10) and in a further step (79) the correction data (55) are established for the correction of the eye (10) on the basis of the adaptive data (35).

8. Method according to the previous claim,
**characterized in that**
additionally aberration data (85) are established which correspond to the objective aberration of the eye (10) and the correction data (55) are established on the basis of the adaptive data (35) and the aberration data (85).

9. Method according to one of the previous method claims,
**characterized in that**
the adaptive data (35) for a subjectively optimum correction adjustment of the eye (10) are obtained by a subjective assessment by the patient of test patterns (45) modified by changing the mirror position of the mirrors (31) of the adaptive lens system (30).

## Revendications

1. Dispositif (1) pour la détermination de données de correction (55) pour la correction des aberrations d'un oeil (10) d'un patient, comprenant un système optique (20), une optique adaptive (30), un dispositif pour la représentation d'images de test (40) qui sont projetés dans l'oeil aux moyens de l'optique adaptive (30 et du système optique (20), et un dispositif de commande(50) pour l'optique adaptive ; pouvant ajouter ainsi des aberrations commandées sur la projection des images de test sur la base d'indications subjectives du patient, lesquelles aberrations compensent les aberrations de l'oeil.

2. Dispositif (1) selon la revendication 1, **caractérisé en ce que** l'optique (30) adaptive se compose d'une multitude de miroirs (31) susceptibles d'être positionnés individuellement.

3. Dispositif (1) selon une des revendications précédentes, **caractérisé en ce que** l'optique (30) adaptive est formée en tant que segment sphérique.

4. Dispositif (1) selon une des revendications précédentes **caractérisé en ce que** l'optique (30) adaptive est susceptible d'être orientée par un premier dispositif de commande (51).

5. Dispositif (1) selon une des revendications précédentes, **caractérisé en ce que** le dispositif (1) comprend en outre :
un dispositif de mesure de l'aberration (80) pour la détermination de données d'aberration (85) qui correspondent à l'aberration objective de l'oeil (10).

6. Dispositif (1) selon une des revendications précédentes, **caractérisé en ce que** les données de correction (55) sont susceptibles d'être déterminées pour la correction des aberrations de l'oeil (10) par un deuxième dispositif de commande (52) sur la base des données adaptives (35) qui correspondent à l'optique adaptive ou aux données adaptives (35) et aux données d'aberration (85).

7. Procédé pour la détermination de données de correction (55) pour la correction des aberrations d'un oeil (10), **caractérisé en ce que** dans une première étape (73) des données de correction (35) sont transmises au moyen d'un dispositif conforme à la revendication 1 pour un ajustement optimal subjectif de l'oeil (10), et **en ce que** dans une étape (79) supplémentaire, on transmet les données de correction (55) pour la correction de l'oeil (10) sur la base des données adaptives (35).

8. Procédé selon la revendication précédente, **caractérisé en ce qu'**on transmet en plus, des données d'aberration (85) qui correspondent à l'aberration objective de l'oeil (10) et **en ce que** les données de correction (55) sont transmises sur la base des données adaptives (35) et des données d'aberration (85).

9. Procédé selon une des revendications de procédé précédentes, **caractérisé en ce que** les données adaptives (35) peuvent être obtenues par le patient pour un ajustement optimal subjectif de l'oeil (10) par une évaluation subjective d'images de test (45) modifiées par le changement de la position du miroir (31) de l'optique (30) adaptive.
